# EUROPEAN PATENT APPLICATION

(11) **EP 3 189 812 A1**
(43) Date of publication of application: **12.07.2017**
(21) Application number: 17150587.8
(22) Date of filing: 08.11.2013
(51) Int. Cl.: A61F 2/28, A61K 9/50, A61K 31/19

(54) **ELUTION OF PLATELET RICH PLASMA COMPONENTS FROM COATED DEVICES**

(30) Priority: 19.11.2012 US 201213680251
(62) Divisional of application: 13815600.5
(71) Applicant: Wisconsin Alumni Research Foundation, Madison, WI 53726 (US)
(72) Inventor: MURPHY, William L., Waunakee, WI 53597 (US); VANDERBY, Ray, Madison, WI 53705 (US); BAER, Geoffrey, Verona, WI 53593 (US); GRAF, Ben K., Madison, WI 53726 (US); LEE, Jae Sung, Middleton, WI 53562 (US); CHAMBERLAIN, Connie, Monona, WI 53714 (US)
(74) Representative: Beacham, Annabel Rose

(57) **Abstract**

Methods for selectively eluting a biological molecule from mineral coated devices are disclosed. The mineral coated devices allow for the isolation and delivery of a biological molecule obtained from the same subject to avoid the safety concerns of current biological therapies obtained by recombinant methods and purification from animal sources.

## Description

### STATEMENT OF GOVERNMENT SUPPORT

This invention was made with government support under AR059916 and HL093282 awarded by the National Institutes of Health. The government has certain rights in the invention.

### BACKGROUND OF THE DISCLOSURE

The present disclosure relates generally to medical devices. More particularly, the present disclosure relates to medical devices having a mineral coated substrate and an autologous biological molecule. The present disclosure further relates to methods for selectively isolating a biological molecule from a bodily fluid using the coated devices and methods for selectively eluting a biological molecule from the coated devices.

The delivery of biological molecules ("biologics") such as growth factors to promote musculoskeletal healing has become a popular approach in industry, with the market for orthopedic growth factors, for example, nearly quadrupling between 2003 and 2008. One delivery strategy involves embedding biologics within collagen sponges for insertion into a tissue defect. This strategy has been used clinically for delivery of the biologic BMP2 and is under development for delivery of other emerging biologics, such as BMP12.

Despite the clinical success of biologics, there are significant limitations related to their delivery. For example, the materials that serve as carriers for biologics, such as collagen sponges, are often inappropriate for orthopedic applications because they do not seamlessly incorporate into standard clinical procedures, and thus, require adoption and training of the medical practitioner. In addition, biologic molecules may quickly diffuse away from carrier materials and may rapidly degrade *in vivo* (e.g., _{t1/2} of BMP2 ∼ minutes), which results in limited bioavailability and a need to deliver large doses of the biological molecules. These large doses may be costly and may present a significant safety concern in the clinical orthopedics community, as they have led to edema and ectopic bone formation in multiple recent clinical studies. Thus, there are significant challenges associated with developing biologics for clinical applications.

Blood is a biological therapy that may be used for whole blood transfusions or making medications. Medications produced from specific portions of the whole blood may be, for example, plasma, platelets, red blood cells, and white blood cells.

Allogeneic (or homologous) blood transfusion uses blood that is collected from a blood donor and is used for the transfusion of another subject. A specific blood type must be matched for safe transfusion. Another common blood donation practice is for a subject to have blood withdrawn in anticipation of needing blood (i.e., self-donation). If, for example, a subject is planning to undergo surgery where a blood transfusion may be necessary, the subject may have blood withdrawn and stored prior to the procedure. Autologous donation may eliminate reactions due to donor-recipient incompatibility and may preclude exposure to transfusion transmitted infection.

Use of platelet rich plasma therapy is an emerging biologic treatment that may influence the healing of tissues. Platelet rich plasma ("PRP") is blood plasma that has been enriched with platelets. Platelet enrichment involves the collection of whole blood that is anticoagulated before undergoing centrifugation to separate PRP from platelet-poor plasma and red blood cells. In humans, the baseline platelet concentration of whole blood is about 160-370 k/µl. PRP contains about 4-10X concentration of baseline platelet concentration. The healing influence of PRP may be attributed to the supraphysiological concentrations of growth factors that are released by activated platelets. Many of the growth factors contained in PRP have been identified as possible biologics and studies are under way to isolate and develop these growth factors for use as biologics.

Recent studies indicate that PRP may accelerate healing, especially in tissues having a poor blood supply. For example, PRP administration improved filling and biomechanical testing of partial anterior cruciate ligament tears. PRP administration has also been shown to increase failure force for Achilles tendon injury and stimulate the development of new bone and tendon in infraspinatus model. Clinical use of PRP has produced promising, but inconsistent results due to the broad variability in the production of PRP by various concentrating equipment and techniques, as well as individual variability in the platelet concentration of plasma.

The preparation and use of biologics, especially those such as blood and blood-derived products, may involve a cumbersome regulatory path. Many growth factors used as biologics are prepared using recombinant protein expression methods, which must undergo regulatory approval. For example, approval of biologics by the U.S. Food and Drug Administration and the European Medicines Agency involves showing the safety, purity, potency and efficacy of a biologic. Blood used for transfusions also undergoes a significant battery of tests to avoid the transmission of blood-borne pathogens.

Accordingly, there exists a need to develop materials and methods for isolating biological molecules such as blood and blood components for therapeutic applications.

### SUMMARY OF THE DISCLOSURE

The present disclosure relates generally to materials and methods for isolating and delivering biological molecules from bodily fluids. More particularly, the present disclosure relates to coated devices that can deliver biological molecules isolated from bodily fluids such as, for example, PRP and PRP components. The present disclosure also relates to methods for isolating and eluting biological molecules from bodily fluids. The coated devices and methods may be used in the operating room, for example, prior to or during a surgical procedure, to isolate biological molecules from a bodily fluid from a patient.

The coated devices and methods offer the possibility of selecting specific biological molecules from bodily fluids such as, for example, blood and blood-derived solutions that may be obtained intraoperatively. Moreover, because the biological molecules may be autologous biological molecules, the dosing and regulatory issues facing current biological molecules may be mitigated.

In one aspect, the present disclosure is directed to a coated device for delivering an autologous biological molecule having a mineral coating on a substrate and an autologous biological molecule.

In another aspect, the present disclosure is directed to a method for selectively isolating a biological molecule from a bodily fluid. The method includes preparing a coated device having a mineral coating on a substrate. The coated device is then incubated with a bodily fluid comprising a biological molecule, wherein the bodily fluid further comprises an ionic buffer.

In another aspect, the present disclosure is directed to a method for selectively eluting a biological molecule from a coated device. The method includes preparing a coated device having a mineral coating on a substrate; incubating the coated device with a bodily fluid; and eluting the biological molecule from the coated device.

### BRIEF DESCRIPTION OF THE DRAWINGS

The disclosure will be better understood, and features, aspects and advantages other than those set forth above will become apparent when consideration is given to the following detailed description thereof. Such detailed description makes reference to the following drawings, wherein:
FIG. 1A is a scanning electron micrograph of a mineralized poly lactide glycolide (PLG) film as discussed in Example 1.
FIG. 1B is a scanning electron micrograph of a mineralized PLG film at a higher magnification as discussed in Example 1.
FIG. 2 is a schematic depicting binding of platelet rich plasma (PRP) components to mineralized PLG films as analyzed in Example 1.
FIG. 3A is a graph illustrating the total protein concentration of diluted PRP incubated with the mineralized PLG film as analyzed in Example 1.
FIG. 3B is a scaled up version of the graph shown in FIG. 3A illustrating the total protein concentration of the 10 dilution incubated with the mineralized PLG film as analyzed in Example 1.
FIG. 4 is a graph illustrating the time- and dose-dependent changes in PRP protein binding to the mineralized PLG films as analyzed in Example 1.
FIG. 5 is a schematic depicting the selective elution of PRP as analyzed in Example 2.
FIG. 6 is a graph illustrating the amount of protein eluted from mineral coated wells after exposure to varying PO₄ concentrations for 15 minutes as analyzed in Example 2.
FIG. 7 is a graph illustrating the amount of protein eluted from mineral coated wells after exposure to varying PO₄ concentrations for 60 minutes as analyzed in Example 2.
FIG. 8 is a graph illustrating the amount of protein eluted from mineral coated wells after exposure to varying PO₄ concentrations for 90 minutes as analyzed in Example 2.
FIG. 9 is a schematic depicting the selective elution of PRP as analyzed in Example 3.
FIG. 10A is a graph illustrating the amount of protein eluted from mineral coated wells after a time point of less than 5 minutes using varying PO₄ concentrations as analyzed in Example 3.
FIG. 10B is a graph illustrating the amount of protein bound to mineral coated wells after exposure to varying PO₄ concentrations as analyzed in Example 3.
FIG. 11A is a graph illustrating the amount of protein eluted from mineral coated wells after a time point of 30 minutes using varying PO₄ concentrations as analyzed in Example 3.
FIG. 11B is a graph illustrating the amount of protein bound to mineral coated wells after exposure to varying PO₄ concentrations as analyzed in Example 3.
FIG. 12A is a graph illustrating the amount of protein eluted from mineral coated wells after a time point of 90 minutes using varying PO₄ concentrations as analyzed in Example 3.
FIG. 12B is a graph illustrating the amount of protein bound to mineral coated wells after exposure to varying PO₄ concentrations as analyzed in Example 3.
FIG. 14 is a graph illustrating the amount of protein bound to a mineralized PLG film coating after a 15 minute concomitant exposure to PRP and PO₄ buffer as analyzed in Example 4.
FIG. 15 is a graph illustrating the amount of protein bound to a mineralized PLG film after a 30 minute concomitant exposure to PRP and PO₄ buffer as analyzed in Example 4.
FIG. 16 is a graph illustrating the amount of protein bound to a mineralized PLG film after a 90 minute concomitant exposure to PRP and PO₄ buffer as analyzed in Example 4.
FIG. 17 is a schematic depicting the selective elution of bovine serum albumin (BSA) as analyzed in Example 5.
FIG. 18A is a graph illustrating the amount of BSA eluted from a mineralized PLG film after exposure to varying PO₄ concentrations for less than 5 minutes as analyzed in Example 5.
FIG. 18B is a graph illustrating the amount of BSA bound to a mineralized PLG film after exposure to varying PO₄ concentrations for less than 5 minutes as analyzed in Example 5.
FIG. 19A is a graph illustrating the amount of BSA eluted from a mineralized PLG film after exposure to varying PO₄ concentrations for 15 minutes as analyzed in Example 5.
FIG. 19B is a graph illustrating the amount of BSA bound to a mineralized PLG film after exposure to varying PO₄ concentrations for 15 minutes as analyzed in Example 5.
FIG. 19C is a graph illustrating the relationship between bound and unbound BSA after a 15 minute exposure to varying PO₄ concentrations as analyzed in Example 5.

While the disclosure is susceptible to various modifications and alternative forms, specific embodiments thereof have been shown by way of example in the drawings and are herein described below in detail. It should be understood, however, that the description of specific embodiments is not intended to limit the disclosure to cover all modifications, equivalents and alternatives falling within the spirit and scope of the disclosure as defined by the appended claims.

### DETAILED DESCRIPTION

Unless defined otherwise, all technical and scientific terms used herein have the same meaning as commonly understood by one of ordinary skill in the art to which the disclosure belongs. Although any methods and materials similar to or equivalent to those described herein may be used in the practice or testing of the present disclosure, the preferred materials and methods are described below.

In accordance with the present disclosure, coated devices and methods for selectively binding and eluting biological molecules from bodily fluids using the coated devices have been discovered. Methods using coated devices as disclosed herein provide a high throughput platform for selectively binding a desired biological molecule as well as a high throughput platform for determining a desired release profile of a biological molecule from the coated device. Significantly, the coated device having an autologous biological molecule of the present disclosure permits the delivery of a biological molecule obtained from the same subject. This feature avoids significant safety concerns with biological molecules prepared using traditional methods such as, for example, recombinant methods and isolation methods from animal sources. The methods further allow for the selective isolation and selective elution of specific biological molecules in a bodily fluid such that specific biological molecules may be delivered to a subject from the coated devices of the present disclosure.

### Coated Devices with a Mineralized Substrate and an Autologous Biological Molecule

In one aspect, the present disclosure is directed to a coated device for delivering an autologous biological molecule. The coated device has a mineral coating on a substrate and an autologous biological molecule attached thereto. Suitable coated devices may be, for example, an orthopedic device, a particle, a film, a dish, a plate, and a suture. Particularly suitable orthopedic devices may be, for example, an arrow, a barb, a tack, an anchor, a nail, a pin, a screw, a staple, a plate, and combinations thereof. Particularly suitable particles may be, for example, agarose beads, latex beads, magnetic beads, and combinations thereof. Particularly suitable plates may be, for example, microtiter plates having, for example, 6, 14, 96, or more sample wells.

The coated device includes a mineral coating on the surface of a substrate. Suitable mineral coatings are made from mineral forming materials such as, for example, calcium, phosphate, carbonate, and combinations thereof. More particularly, as described more fully herein, the mineral coatings may be formed on the substrate by surface hydrolyzing the substrate under alkaline conditions. After surface hydrolyzing, the substrate is incubated in a modified simulated body fluid (mSBF) containing a suitable mineral-forming material. Suitable substrates for use with the coatings may be, for example, a poly(α-hydroxy ester). Particularly suitable poly(α-hydroxy esters) may be, for example, poly(L-lactide), poly(lactide-co-glycolide), poly(ε-caprolactone), and combinations thereof.

Attached to the coating, is an autologous biological molecule. The autologous biological molecule is obtained from an autologous bodily fluid. The term "autologous bodily fluid" is used herein to refer to a bodily fluid that is obtained from a subject and used as the source of the autologous biological molecule, which is attached to the coated device that is returned to the same subject. Thus, the subject is both the donor and recipient of the autologous biological molecule. For example, if the autologous bodily fluid is platelet rich plasma (PRP), the PRP is obtained from a subject and is then incubated with the coated device according to the method described herein to bind an autologous biological molecule contained within the subject's own PRP. Suitable autologous bodily fluids may be, for example, whole blood, serum, plasma, platelet rich plasma, bone marrow, cerebrospinal fluid, urine, synovial fluid, and combinations thereof.

Suitable autologous biological molecules obtained from the autologous bodily fluids may be proteins, for example. Particularly suitable proteins may be, for example, basic proteins. The term "basic protein" is used herein according to its ordinary meaning as understood by those skilled in the art to refer to the category of proteins that have a high isoelectric point (pI of from about 7.1 to about 14), and therefore, tend to be positively charged at physiological pH (∼7.4). By contrast, the term "acidic protein" is used herein according to its ordinary meaning as understood by those skilled in the art to refer to the category of proteins that have a low isoelectric point (pI of from about 0 to about 6.9), and therefore tend to be negatively charged at physiological pH (∼7.4). Particularly suitable basic proteins may be, for example, growth factors.

Suitable growth factors may be, for example, bone morphogenic proteins (BMPs), connective tissue growth factors, epidermal growth factors, fibroblast growth factors (FGFs), insulin-like growth factors, interleukin, keratinocyte growth factors, platelet-derived growth factor (PDGFs), transforming growth factors (TGFs), vascular endothelial growth factors (VEGF), nerve growth factor (NGF), hepatocyte growth factor (HGF), tumor necrosis factors (TNF), interferons (IFN), and combinations thereof. Specific suitable growth factors may be, for example, BMP1, BMP2, BMP3, BMP4, BMP5, BMP6, BMP7, BMP8a, EGF, PDGFA, PDGFB, PDGFC, PDGFD, PDGFAB, VEGF-A, placenta growth factor (PIGF), VEGF-B, VEGF-C, VEGF-D, TGF-f31, TGF-f32, TGF-f33, AMH, ARTN, GDF1, GDF2, GDF3, GDF3A, GDF5, GDF6, GDF7, GDF8, GDF9, GDF10, GDF11, GDF15, GDFN, INHA, INHBA, INHBB, INHBC, INHBE, LEFTY1, LEFTY2, MSTN, NODAL, NRTN, PSPN, IL-1, IL-2, IL-4, IL-5, IL-6, IL-12, IL-13, IL-21, IL-23, IL-17, IFN-α, IFN-γ, TNF-α, TNF-f3, FGF1, FGF2, FGF3, and FGF4.

The resulting coated device having an autologous biological molecule bound thereto may then be administered, for example, back into the same subject that served as the donor of the autologous bodily fluid. For example, the coated device may be implanted in a subject to deliver the autologous biological molecule to the subject.

Because the coated device of the present disclosure has an autologous biological molecule, the coated device allows for the delivery of the autologous biological molecule without the concerns associated with using biological molecules obtained by traditional methods such as, for example, recombinant methods and from animal sources. Also, because the coated devices have a mineral coating that is degradable, delivery of the autologous biological molecules may be controlled such as through controlled elution of the biological molecule from the mineralized coating and/or controlled degradation of the mineral coating. As the mineral coating degrades, the attached autologous biological molecule is released from the coated device. Moreover, the autologous biological molecule attached to the coated device may stimulate repair and/or growth by stimulating cells surrounding or recruited to the area containing the coated device. Additionally, therapeutically effective amounts of the autologous biological molecule may be administered as the concentration of the autologous biological molecules on the coated device may be controlled.

### Methods for selectively isolating a biological molecule from a bodily fluid

In one aspect, the present disclosure is directed to a method for selectively isolating a biological molecule from a bodily fluid. The method includes preparing a coated device comprising a mineral coating on a substrate and incubating the coated device with a bodily fluid comprising a biological molecule, wherein the bodily fluid further comprises an ionic buffer.

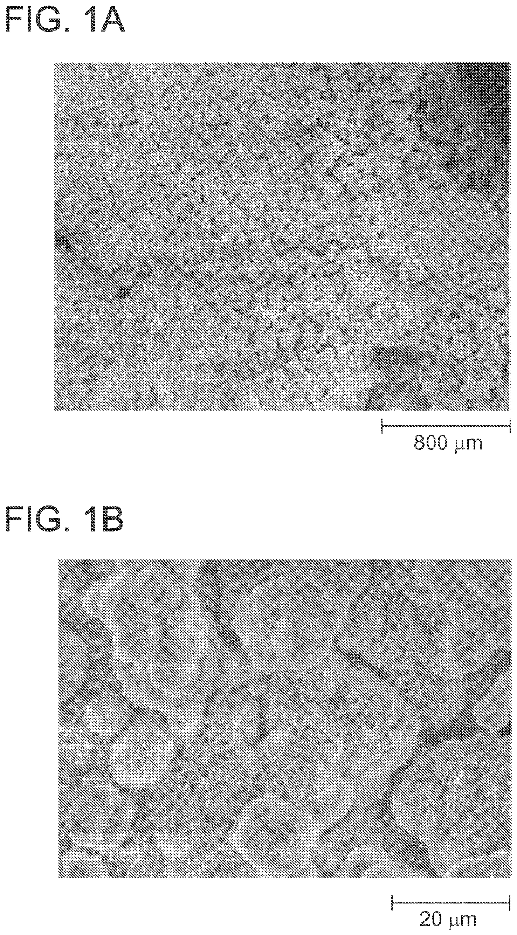

To prepare the coated device, a mineral coating may first be formed on the substrate using methods described in U.S. Patent Application Pub. No. 20080095817, U.S. Patent No. 8,075,562, and U.S. Patent Application Pub. No. 20110305760, which are hereby incorporated by reference to the extent they are consistent herewith. For example, the mineral coating may be formed by surface hydrolyzing a substrate under alkaline conditions such as, for example, NaOH, followed by incubation in a modified simulated body fluid (mSBF) at a physiologic temperature and pH 6.8 for mineral nucleation and growth. The mSBF solution contains mineral-forming ions, including calcium, phosphate, carbonate, and combinations thereof. The resulting coating may be any suitable coating material containing calcium, phosphate and carbonate, such as, for example, hydroxyapatite (HAP), α-tricalcium phosphate (α-TCP), f33-tricalcium phosphate (f33-TCP), amorphous calcium phosphate, dicalcium phosphate, octacalcium phosphate, and calcium carbonate. Further, the coating formed on the substrate develops as a porous mineral coating (see FIGS. 1A and 1B). Although porous mineral coatings are particularly suitable, the mineral coatings may also be nonporous.

As described above, suitable substrates may be, for example, a poly(α-hydroxy ester). Particularly suitable poly(α-hydroxy esters) may be, for example, poly(L-lactide), poly(lactide-co-glycolide), poly(E-caprolactone), and combinations thereof.

The coated device having the mineral coated substrate is then incubated with a bodily fluid including one or more desired biological molecules. In one embodiment, the bodily fluid may include autologous bodily fluid as described above.

Alternatively, the bodily fluid is a heterologous bodily fluid. As used herein, the term "heterologous bodily fluid" (i.e., non-autologous solution) refers to a bodily fluid that is obtained from one subject and used in the method to prepare a coated device having a biological molecule attached thereto. The resulting coated device is then used for treating a different subject (i.e., not the subject that donated the bodily fluid). Thus, a subject who is the donor of the heterologous bodily fluid used in the method is different from a subject who is the recipient of the coated device. The term "heterologous bodily fluid" also refers to a bodily fluid that is obtained from a different species of animal, which is then used in the method for isolating a biological molecule of the present disclosure. For example, a bodily fluid such as PRP from a sheep may be used to isolate a biological molecule from the sheep PRP, which is then used for a non-sheep animal as the recipient.

Suitable autologous and heterologous bodily fluids may be, for example, whole blood, serum, plasma, platelet rich plasma, bone marrow, cerebrospinal fluid, urine, synovial fluid, and combinations thereof.

The bodily fluid further includes an ionic buffer. Advantageously, including an ionic buffer in the bodily fluid allows for the selective binding of biological molecules to the coated device. The ionic buffer may be added to the bodily fluid while the coated device is incubated. Without being bound by theory, adding an ionic buffer to the bodily fluid during incubation with the coated device may interfere with the formation of electrostatic interactions between the mineral coating and the biological molecule contained in the bodily fluid. For example, if the ionic buffer added to the bodily fluid is high enough to prevent the formation of electrostatic interactions between a biological molecule and the mineral coating, the biological molecule may not bind to the mineral coating. Thus, the ionic strength of the buffer that is added to the bodily fluid influences binding of a biological molecule to the mineral coating. For example, the ionic buffer may influence binding of a biological molecule such that the biological molecule does not interact at all to the coated device, weakly interacts with the coated device, and/or strongly interacts with the coated device.

Suitable ionic buffers that may be used in the method for selectively isolating a biological molecule from a bodily fluid may be any ionic buffer that disrupts, interferes with, and/or competes with the electrostatic interaction between the biological molecule and the mineral of the mineral coating on the coated device. Particularly suitable ionic buffers may be, for example, phosphate buffers, sodium chloride buffers, magnesium chloride buffers, calcium chloride buffers, sodium fluoride buffers, and combinations thereof. Particularly suitable phosphate buffers may have a phosphate concentration up to, and including, 0.5 M phosphate, and including from about 0.001 M to 0.5 M phosphate. Particularly suitable sodium chloride buffers may have a sodium chloride concentration up to, and including, 0.2 M sodium chloride. Particularly suitable magnesium chloride buffers may have a magnesium chloride concentration up to, and including, 5.0 M magnesium chloride. Particularly suitable calcium chloride buffers may have a calcium chloride concentration up to, and including, 5.0 M calcium chloride. Particularly, suitable sodium fluoride buffers may have a sodium fluoride concentration up to, and including, 0.4 M sodium fluoride.

Suitable ionic buffers may be, for example, buffers having varying pH ranges. Suitable pH ionic buffers may have a pH range of from about 6.4 to about 7.8.

The coated device having the mineral coated substrate is incubated with the bodily fluid for a sufficient period of time to allow attachment of a biological molecule to the mineral coating of the substrate. Suitable time to allow the biological molecule to attach to the mineral coating of the substrate may be, for example, from less than a minute to about 120 minutes. The biological molecule attaches to the mineral coating by electrostatic interactions.

The method may further include washing the coated device to remove components contained within the bodily fluid that do not bind to the mineral coating. Washing may remove serum albumin, for example.

The coated device may be washed using any suitable washing solution. Suitable washing solutions may be, for example, HEPES (4-(2-hydroxyethyl)-1-piperazineethanesulfonic acid), saline, 0.001 M phosphate buffer, double distilled water, and combinations thereof.

Attachment of a biological molecule may be monitored using methods known to those skilled in the art. For example, the total protein concentration of the bodily fluid before and after incubation with the coated device may be monitored using a BCA (bicinchoninic acid) assay. Other suitable assays that may be used to monitor attachment and/or elution may be, for example, ELISA, Western blot, 1D and 2D SDS-PAGE, non-equilibrium pH gel electrophoresis (NEPHGE), AGILENTTM protein analysis, and combinations thereof.

The coated devices resulting from the methods may be coated devices having a mineral coating on a substrate and a heterologous or autologous biological molecule attached thereto. If the resultant coated device is one having a heterologous biological molecule, the coated device is used for a subject that is different from the subject that donated the bodily fluid (the heterologous bodily fluid) used in the method to selectively isolate the biological molecule. Alternatively, if the resultant coated device is one having an autologous biological molecule, the coated device is used for a subject that also was the subject that donated the bodily fluid (the autologous bodily fluid) used in the method to selectively isolate the biological molecule.

The resultant coated device may be administered to a subject. The resultant coated device may be administered, for example, as an implant. For example, the resultant coated device may be implanted in a subject to deliver the biological molecule to a subject.

The resultant coated device having an autologous biological molecule allows for the delivery of the autologous biological molecule without the concerns associated with using biological molecules obtained by traditional methods such as, for example, recombinant methods and isolation methods from animal sources. Thus, the resultant coated device having an autologous biological molecule can avoid regulatory hurdles and safety issues associated with biological molecules obtained from sources other than from the subject's own bodily fluids. The resultant coated device having a heterologous biological molecule allows for the delivery of the heterologous biological molecule under conditions where the recipient subject may not have a sufficient amount of a biological molecule such that the recipient can also be the donor of the bodily fluid used in the method. Additionally, a resultant coated device having a heterologous biological molecule may be suitable for use in a veterinary setting, where one donor subject may be used to provide the bodily fluid used in the method for preparing multiple coated devices or where having an autologous biological molecule is not desired.

Because the coated devices have a mineral coating that is degradable, delivery of the autologous biological molecule and the heterologous biological molecule may be controlled such through controlled elution of the biological molecule from the mineral coating as described herein and/or controlled degradation of the mineral coating. As the mineral coating degrades, the attached autologous biological molecule and/or the heterologous biological molecule may be released from the coated device. Additionally, or alternatively, as the ionic concentration of the environment in which the coated device is implanted changes, it may influence the electrostatic interaction between the biological molecule and the mineral coating such that the biological molecule detaches from the mineral coating.

Moreover, the autologous biological molecules and the heterologous biological molecules may stimulate repair and/or growth by stimulating cells surrounding or recruited to the area containing the coated device. Therapeutically effective amounts of the autologous biological molecule and/or the heterologous biological molecule may be administered as the concentration of the autologous biological molecules and the heterologous biological molecule on the coated device may be controlled.

### Methods for selectively eluting a biological molecule from a coated device

In another aspect, the present disclosure is directed to a method for selectively eluting a biological molecule from a coated device. The method includes preparing a coated device having a mineral coating on a substrate; incubating the coated device with a bodily fluid having a biological molecule; and eluting the biological molecule from the coated device. Eluting may remove biological molecules that are attached to the mineral coating on the substrate, and in some embodiments, allows for selectively removing a biological molecule from the coated device.

In one aspect, eluting at least one biological molecule from the coated device includes contacting the coated device with an elution buffer. Suitable elution buffers may be any ion-containing buffer that disrupts the electrostatic interaction between the biological molecule and the mineral of the mineral coating on the coated device. Particularly suitable elution buffers that may be used to elute at least one biological molecule from the coated device may be, for example, phosphate buffers (up to 0.5 M phosphate), sodium chloride buffers (up to0.2 M sodium chloride), magnesium chloride buffers (up to 5.0 M magnesium chloride), calcium chloride buffers (up to 5.0 M calcium chloride), sodium fluoride buffers (up to 0.4 M sodium fluoride), and combinations thereof.

Selective elution of the biological molecule may be performed as a "batch-type" elution in which the coated device is contacted with a particular ionic strength buffer such that elution of the biological molecule occurs at once. Selective elution of the biological molecule may also be performed using a concentration gradient in which the beginning elution is performed using a low ionic strength buffer and continues with increasing ionic strength buffer. The gradient may be performed as a step-wise gradient or as a continuous gradient.

In another aspect, eluting at least one biological molecule from the coated device includes contacting the coated device with a mineral dissolution buffer. The mineral dissolution buffer causes the mineral of the mineral coating to dissolve. As the mineral coating dissolves, a biological molecule that is electrostatically attached to the mineral coating loses its attachment and elutes from the coated device. Suitable mineral dissolution buffers may be any buffer that causes the mineral coating to dissolve.

Particularly suitable mineral dissolution buffers may include phosphoric acid, ethylenediaminetetraacetic acid (EDTA), 0.25 M hydrochloric acid (HCl), 0.25 M sodium hydroxide (NaOH), for example. The mineral dissolution buffer may include phosphoric acid up to, and including, 0.5 M phosphoric acid. The mineral dissolution buffer may include EDTA up to, and including, 20% (w/v) EDTA. The amount of HCl in the mineral dissolution buffer may be up to, and including, 0.25 M HCl.

Elution of a biological molecule may be monitored using methods known to those skilled in the art. For example, the total protein concentration of the bodily fluid before and after incubation with the coated device as well as after the coated device is contacted with an ionic buffer or a mineral dissolution buffer may be monitored using a BCA assay. Other suitable assays that may be used to monitor elution may be, for example, ELISA, Western blot, 1D and 2D SDS-PAGE, non-equilibrium pH gel electrophoresis (NEPHGE), AGILENTTM protein analysis, and combinations thereof.

The disclosure will be more fully understood upon consideration of the following non-limiting Examples.

### EXAMPLES

### EXAMPLE 1

### Mineralized Film and PRP

In this Example, the protein concentration of PRP was determined after incubation with a mineralized poly lactide glycolide (PLG) film.

Specifically, poly lactide glycolide (85:15) was solvent casted into a film. The film was mineralized for 10 days using mSBF to form a mineralized coating on the PLG film (FIG. 1A and FIG. 1B). Blood was collected from sheep and centrifuged at 312 X g and 1248 X g to obtain PRP having a platelet count of 886 k/µl. PRP was subjected to cycles of freeze-thaw to lyse platelets. The resulting PRP was diluted by factors of 1, 10, and 100 in 0.001 M phosphate (PO₄) buffer. The total protein concentration of each PRP dilution was determined by BCA assay.

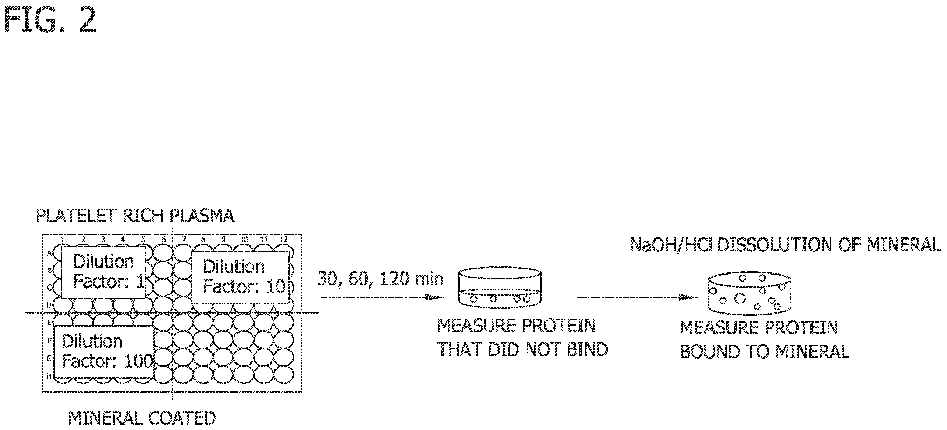

As shown in FIG. 2, mineralized PLG films were incubated with each PRP dilution at 0 minute, 30 minute, 60 minute and 120 minute time points to allow proteins to bind to the mineralized PLG films. After incubation, the films were transferred to a new plate and rinsed with 0.001 M PO₄ buffer to remove unbound protein. The PRP solution from which the films were transferred was collected and used to measure unbound protein. The bound proteins were then eluted from the mineralized PLG films using 0.2 M NaOH and neutralized using 0.2 M HCl in 0.01 M HEPES. Protein concentration of (1) PRP before addition to the mineralized PLG films, (2) PRP after incubation with the mineralized PLG films, and (3) 0.2 M NaOH eluate were determined using a BCA assay.

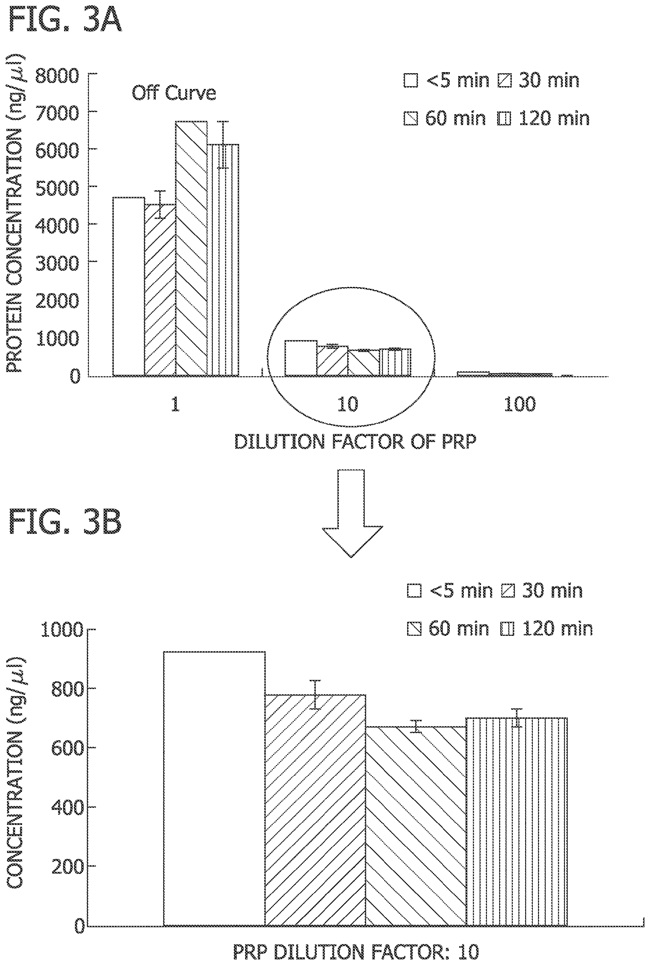

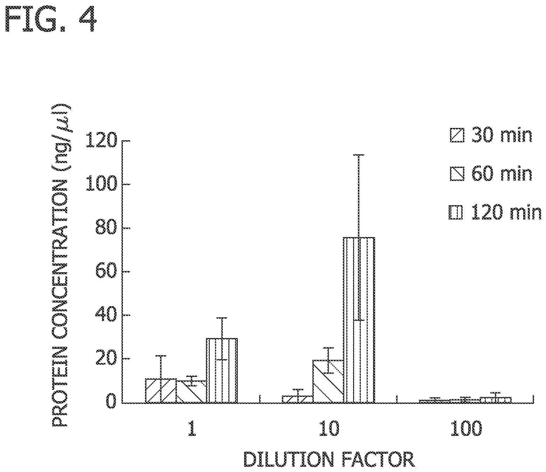

As shown in FIG. 3A and FIG. 3B, the total protein concentration of PRP decreased as the dilution increased. As shown in FIG. 4, incubation of PRP with mineralized PLG films resulted in binding of proteins to the mineralized PLG films. It was also possible to elute the protein that was bound to the mineralized PLG film. Of the three dilutions, the condition that resulted in the most total bound protein was by the mineralized PLG film incubated for 120 minutes with PRP that was diluted by a factor of 10.

### EXAMPLE 2

### Selective Elution of PRP Components

In this Example, elution of proteins from mineralized PLG wells with varying phosphate molarities was determined.

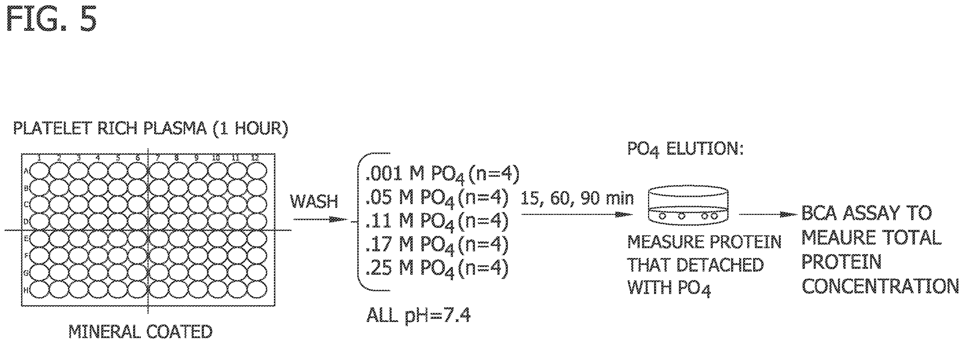

Specifically, wells of a 96-well plate were coated with a mineralized film made as described above. PRP was obtained and subjected to cycles of freeze-thaw to lyse platelets as described above. As shown in FIG. 5, mineralized PLG wells were incubated for 1 hour to allow proteins to bind. Unbound proteins were rinsed off using water. Bound proteins were eluted from the mineralized PLG wells for 15 minute, 60 minute, and 90 minute time points using 0.001 M, 0.05 M, 0.11 M, 0.17 M, and 0.25 M PO₄ (1^{st} Protein Elution) and measured by BCA assay. Proteins that remained bound after the 1^{st} Protein Elution were eluted from the mineralized PLG wells by incubating the mineralized PLG wells using 0.2 M NaOH. The 0.2 M NaOH eluate was neutralized using 0.2 M HCl in 0.01 M HEPES (2^{nd} Protein Elution). The protein concentration of the 2^{nd} Elution was measured using a BCA assay.

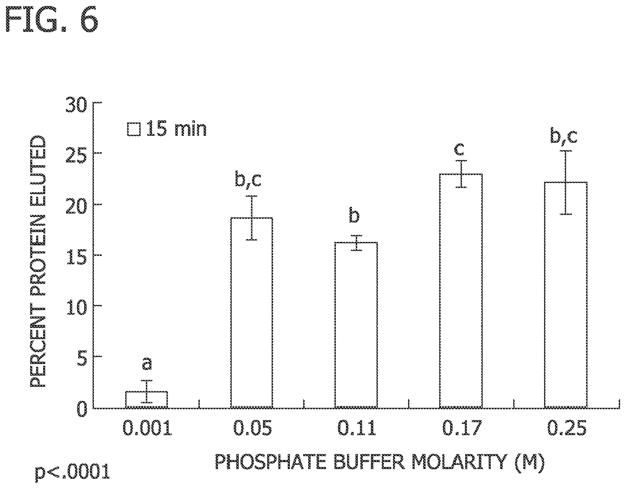

As shown in FIG. 6 for the 15 minute time point, a concentration of 0.001 M PO₄ eluted the least amount of proteins, whereas 0.05 M, 0.11 M, 0.17 M, and 0.25 M PO₄ eluted more proteins.

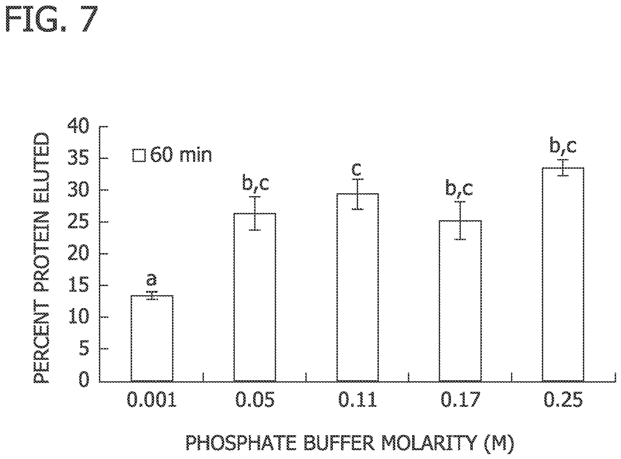

As shown in FIG. 7 for the 60 minute time point, a concentration of 0.001 M PO₄ eluted the least amount of proteins, whereas 0.05 M, 0.11 M, 0.17 M, and 0.25 M PO₄ eluted more proteins.

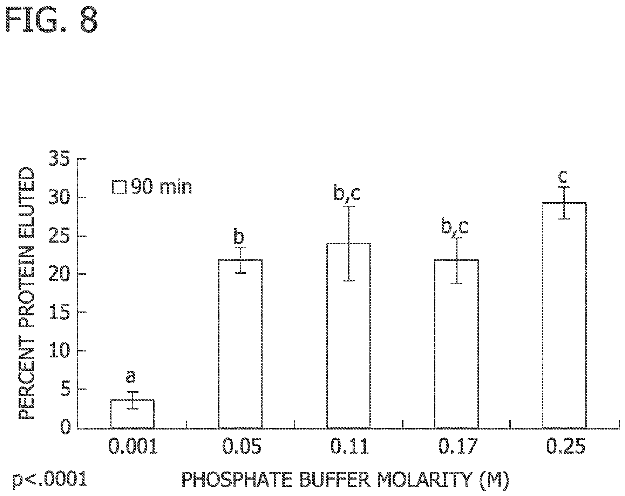

As shown in FIG. 8 for the 90 minute time point, a concentration of 0.001 M PO₄ eluted the least amount of proteins, whereas 0.05 M, 0.11 M, 0.17 M, and 0.25 M PO₄ eluted more proteins.

### EXAMPLE 3

### Selective Elution of PRP Components

In this Example, elution of proteins from mineralized PLG wells with varying phosphate molarities was determined.

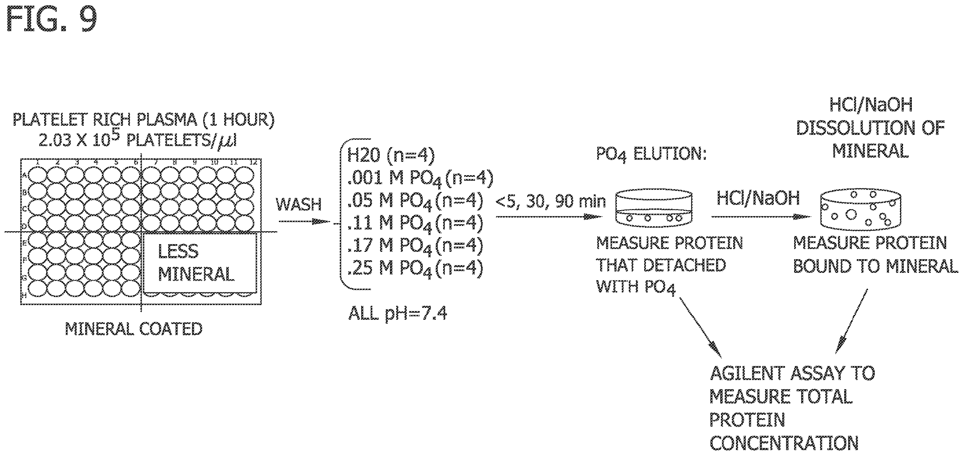

Specifically, PLG wells were mineralized as described above. PRP was obtained and subjected to cycles of freeze-thaw to lyse platelets as described above. As shown in FIG. 9, mineralized PLG wells were incubated for 1 hour to allow proteins to bind. Unbound proteins were rinsed off using water. Bound proteins were eluted from the mineralized PLG wells for less than 5 minutes (buffers were placed into the wells and immediately collected), 30 minutes and 90 minutes using water, 0.001 M, 0.05 M, 0.11 M, 0.17 M, and 0.25 M PO₄ (1^{st} Protein Elution; PO₄ eluted) and measured using an AGILENTTM protein assay. Proteins that remained bound after the 1^{st} Protein Elution (using phosphate buffer) were eluted a second time from the mineralized PLG wells by incubating the mineralized PLG wells using 0.2 M HCl/0.01 M HEPES. The 0.2 M HCl/0.01 M HEPES eluate was neutralized using 0.2 M NaOH (2^{nd} Protein Elution; HCl eluted). The protein concentration of the 2^{nd} Elution was measured using an AGILENTTM assay.

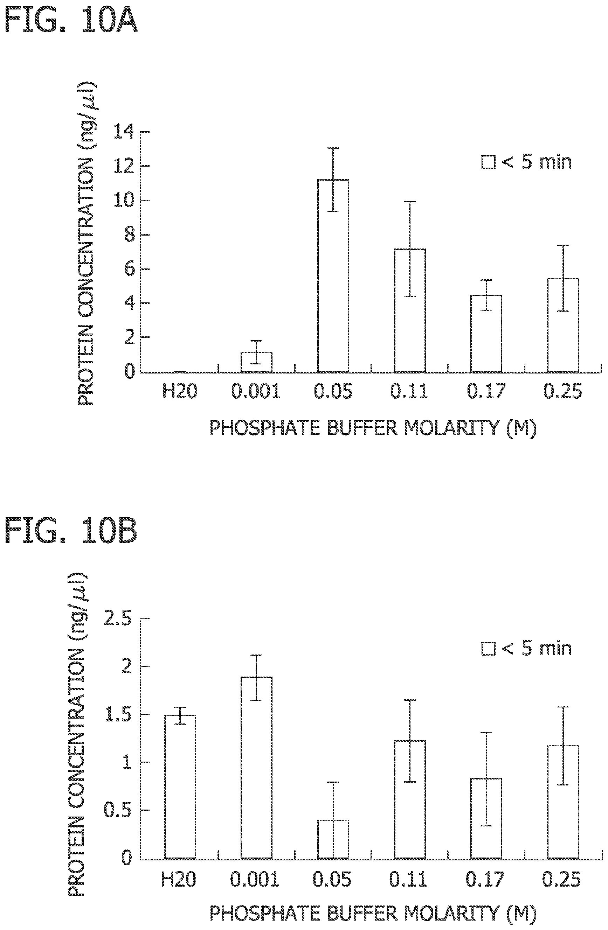

As shown in FIGS. 10A and 10B for the <5 minute time point, most of the protein eluted in the 1^{st} Protein Elution as compared with the percent protein eluted in the 2_{nd} Elution. Additionally, as shown in FIG. 10A, both water and 0.011 M PO₄ eluted the least amount of proteins, whereas 0.05 M, 0.11 M, 0.17 M, and 0.25 M PO₄ eluted more proteins. As shown in FIG. 10B, however, after mineral dissolution via HCl, both water and 0.001 M PO₄ bound the most proteins, whereas 0.05 M, 0.11 M, 0.17 M, and 0.25 M PO₄ bound less proteins.

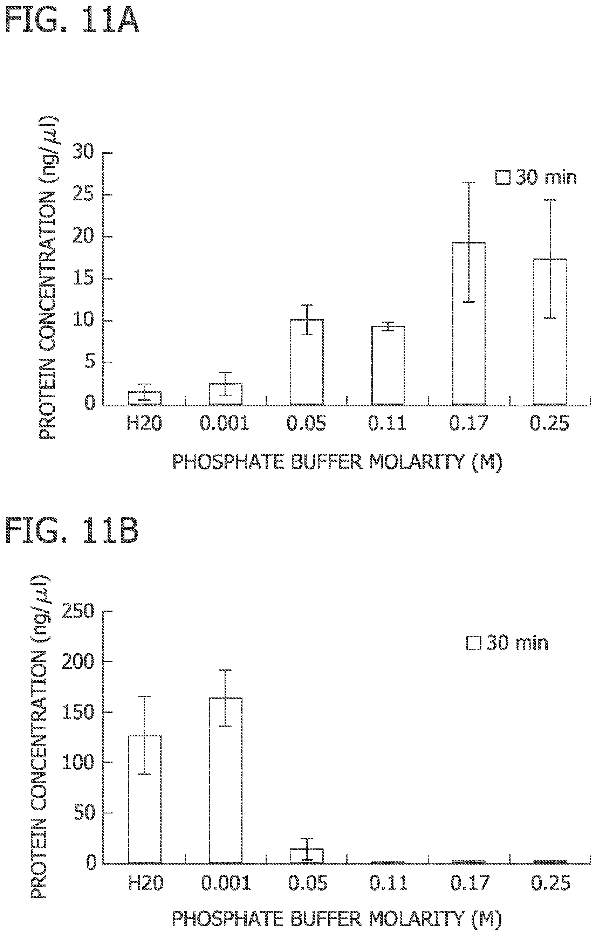

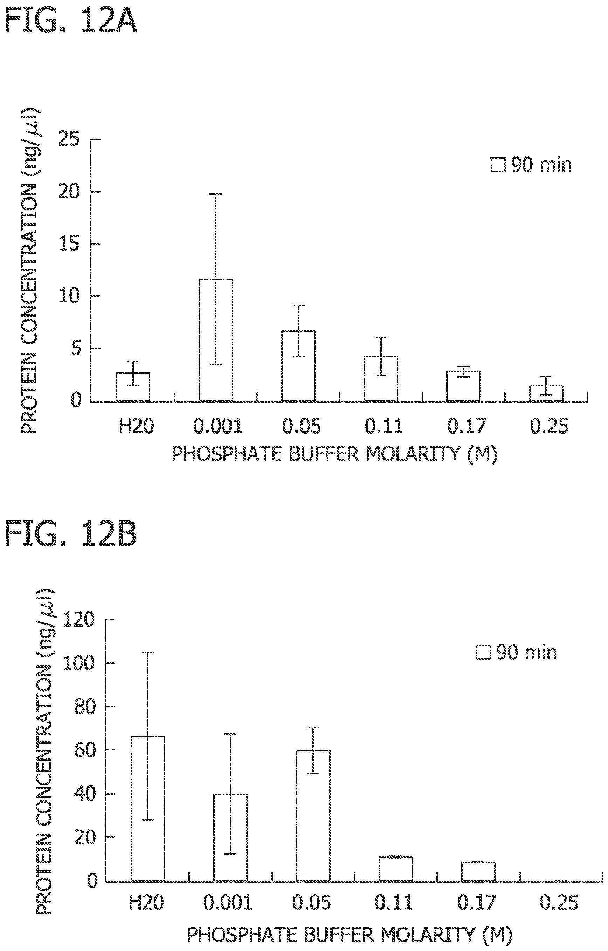

As shown in FIG. 11A for the 30 minute time point, both water and 0.011 M PO₄ eluted the least amount of proteins, whereas 0.05 M, 0.11 M, 0.17 M, and 0.25 M PO₄ eluted more proteins. As shown in FIG. 11B, however, after mineral dissolution via HCl, both water and 0.001 M PO₄ bound the most proteins, whereas 0.05 M, 0.11 M, 0.17 M, and 0.25 M PO₄ bound less proteins. This data also indicated that proteins exposed to PO₄ for 30 minutes have sufficient time to fully elute from the mineral coating. As shown in FIG. 12A for the 90 minute time point, 0.011 M PO₄ eluted the most amount of proteins, whereas 0.05 M, 0.11 M, 0.17 M, and 0.25 M PO₄ eluted less proteins. As shown in FIG. 12B, however, after mineral dissolution via HCl, both water, 0.001 M and 0.05 M PO₄ bound the most proteins, whereas 0.11 M, 0.17 M, and 0.25 M PO₄ bound less proteins. This data also indicated the ability of proteins to bind and release at lesser concentrations from lower density mineral coated wells.

### EXAMPLE 4

### Selective Binding of Proteins from PO₄-modified PRP

In this Example, binding of proteins in PO₄-modified PRP to mineralized PLP films was determined.

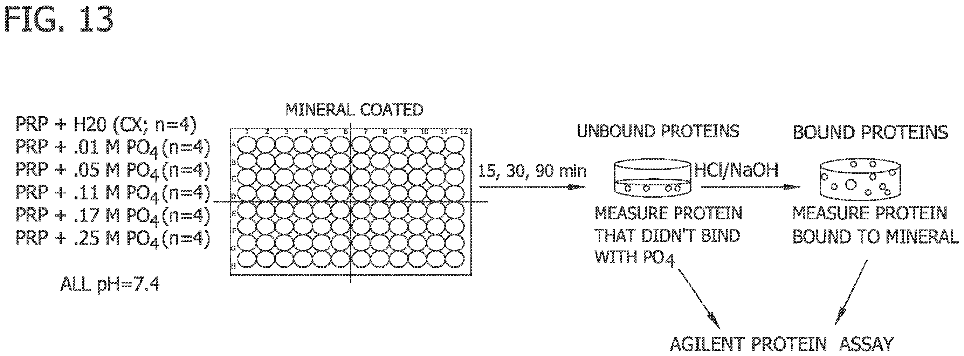

Specifically, PLG films were mineralized as described above. PRP was obtained and subjected to cycles of freeze-thaw to lyse platelets as described above. Varying concentrations of phosphate (0.001 M, 0.05 M, 0.11 M, 0.17 M, and 0.25 M PO₄) were added to the PRP to form the PO₄-modified PRP. No phosphate was added to control PRP ("Cx"). As shown in FIG. 13, mineralized PLP films were incubated with Cx and modified-PRP for 15 minute, 60 minute and 90 minute time points. After the 15 minute, 30 minute and 90 minute incubation periods, the buffer (1^{st} Elution) was collected and analyzed using an AGILENTTM assay to measure total unbound protein. Proteins that bound to the mineralized PLG films were eluted with 0.2 N HCl (2^{nd} Elution) to release bound proteins. The solution was neutralized with 0.2 N NaOH and proteins that selectively bound to the mineralized PLG film were thereby measured using an AGILENTTM assay.

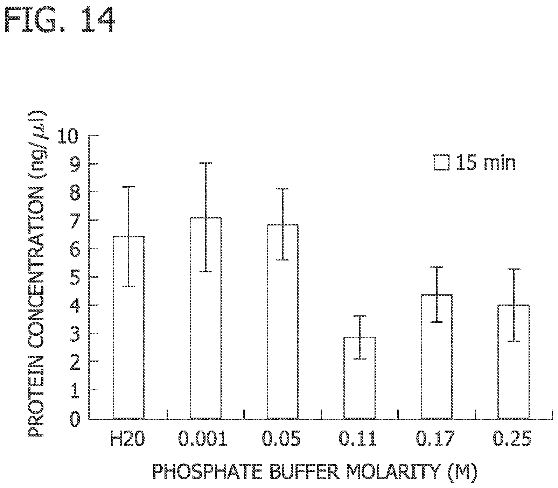

As shown in FIG. 14 for the 15 minute time point, both water, 0.001 M and 0.05 M PO₄ bound the most proteins, whereas 0.11 M, 0.17 M and 0.25 M PO₄ bound less proteins.

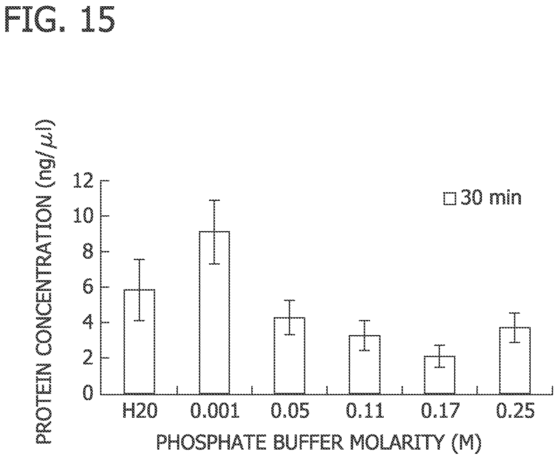

As shown in FIG. 15 for the 30 minute time point, both water and 0.001 M PO₄ bound the most proteins, whereas 0.05 M, 0.11 M, 0.17 M and 0.25 M PO₄ bound less proteins.

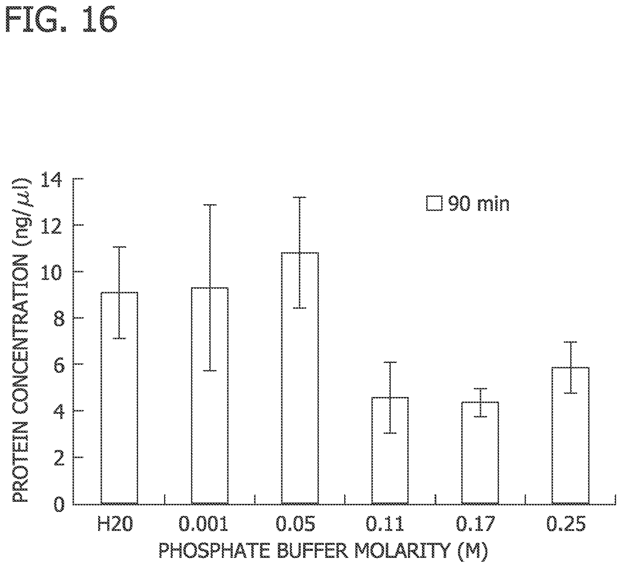

As shown in FIG. 16 for the 90 minute time point, both water and 0.001 M PO₄ bound the most proteins, whereas 0.05 M, 0.11 M, 0.17 M and 0.25 M PO₄ bound less proteins.

### EXAMPLE 5

In this Example, binding of bovine serum albumin (BSA) to mineralized PLG films was determined.

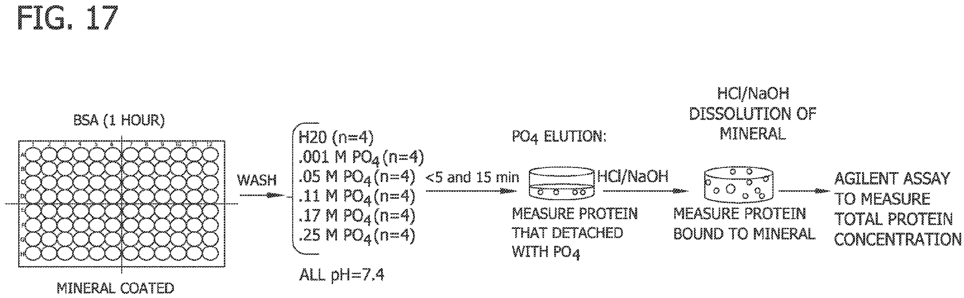

As shown in FIG. 17, BSA was added to mineral coated films and allowed to bind for 1 hour. Unbound BSA was rinsed away. PO₄ buffer at one of the following concentrations, 0.001 M, 0.05 M, 0.11 M, 0.17 M, and 0.25 M, was added to the mineralized films and allowed to incubate for less than 5 minutes (buffer was placed into wells and immediately collected) and at a 15 minute time point. The buffer solution was then collected to measure proteins that eluted from the mineralized film. The mineralized film was then dissolved using 0.2 N HCl to release the bound proteins. The solution was neutralized with 0.2 N NaOH and proteins that selectively bound to the mineralized film were thereby measured using an AGILENTTM assay.

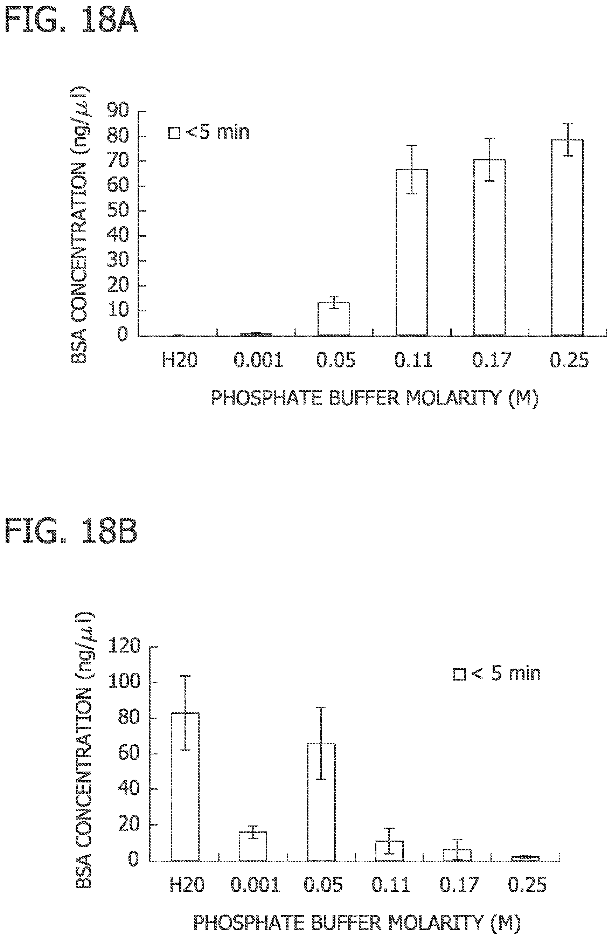

As shown in FIG. 18A, at the less than 5 minute time point, concentrations of 0.001 M and 0.05 M PO₄ eluted the least amount of proteins whereas 0.11 M, 0.17 M, and 0.25 M PO₄ eluted more proteins. As shown in FIG. 18B, water and concentrations of 0.001 M and 0.05 M PO₄ bound the most amount of BSA proteins and 0.11 M, 0.17 M, and 0.25 M PO₄ bound less proteins.

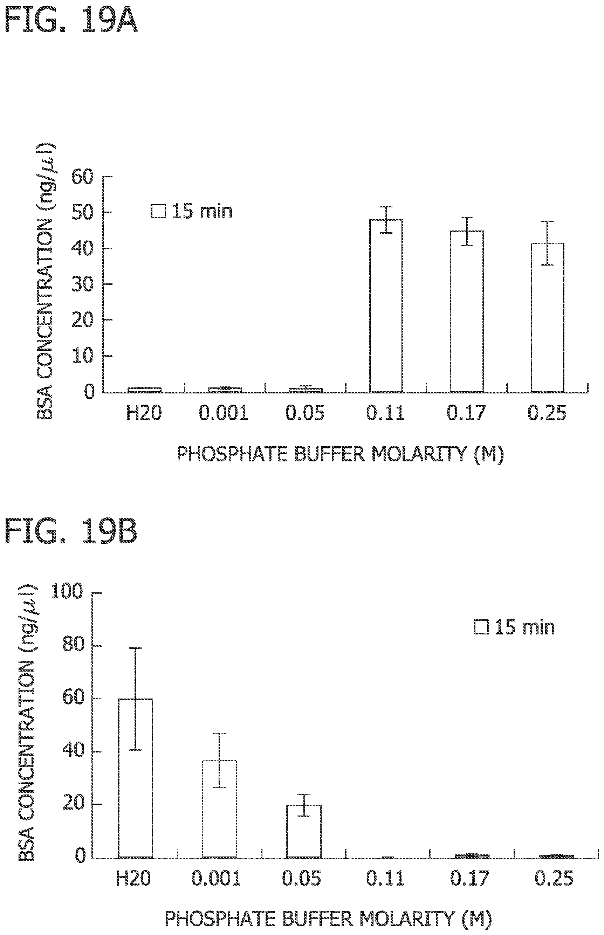

As shown in FIG. 19A, at the 15 minute time point, concentrations of 0.001 M and 0.05 M PO₄ eluted the least amount of proteins whereas 0.11 M, 0.17 M, and 0.25 M PO₄ eluted more proteins. As shown in FIG. 19B, water and concentrations of 0.001 M and 0.05 M PO₄ bound the most amount of BSA proteins and 0.11 M, 0.17 M, and 0.25 M PO₄ bound less proteins.

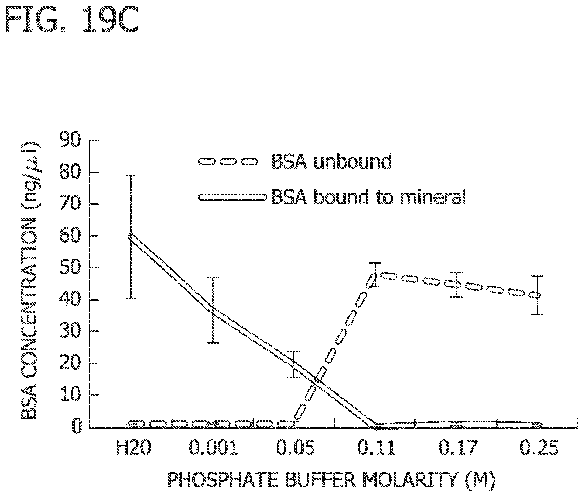

FIG. 19C illustrates the relationship of bound versus unbound BSA after a 15 minute exposure to varying PO₄ concentrations. BSA bound to the mineral coating in a PO₄-dose dependent manner. Combining two different assays, it has been shown that: 1) there is reduced binding to mineral coating with increasing PO₄ molarity (solid line); and 2) there is increased free protein with increasing PO₄ molarity (dotted line). Taken together, these results indicate that albumin binding to mineral is controlled via PO₄ concentration.

The examples described above demonstrate that the mineral coatings and methods offer the ability to select specific biological molecules from bodily fluids that may be obtained intraoperatively. This will allow for the isolation of a specific biological molecule or set of biological molecules from a subject, then delivery of the specific biological molecule back into the same subject or into a different subject.

In view of the above, it will be seen that the several advantages of the disclosure are achieved and other advantageous results attained. As various changes could be made in the above devices and methods without departing from the scope of the disclosure, it is intended that all matter contained in the above description and shown in the accompanying drawings shall be interpreted as illustrative and not in a limiting sense.

When introducing elements of the present disclosure or the various versions, embodiment(s) or aspects thereof, the articles "a", "an", "the" and "said" are intended to mean that there are one or more of the elements. The terms "comprising", "including" and "having" are intended to be inclusive and mean that there may be additional elements other than the listed elements.

Embodiments of the Invention include embodiments 1-6 below:
1. A method for selectively isolating a biological molecule from a bodily fluid, the method comprising:
   preparing a coated device comprising a mineral coating on a substrate;
   incubating the coated device with a bodily fluid comprising a biological molecule,
   wherein the bodily fluid further comprises an ionic buffer.
2. The method of embodiment 1, wherein the bodily fluid is selected from the group consisting of whole blood, serum, plasma, platelet rich plasma, bone marrow, cerebrospinal fluid, urine, synovial fluid, and combinations thereof.
3. The method of embodiment 1, wherein the ionic buffer comprises at least one of phosphate, sodium chloride, magnesium chloride, and calcium chloride.
4. The method of embodiment 3, wherein the ionic buffer comprises up to 0.5 M phosphate.
5. The method of embodiment 1, wherein the substrate comprises a poly(α-hydroxy ester) selected from the group consisting of poly(L-lactide), poly(lactide-co-glycolide), poly(ε-caprolactone), and combinations thereof.
6. The method of embodiment 1, wherein the mineral coating is selected from the group consisting of calcium, phosphate, carbonate, and combinations thereof.

## Claims

1. A method for selectively eluting a biological molecule from a coated device, the method comprising:
preparing a coated device comprising a mineral coating on a substrate;
incubating the coated device with a bodily fluid comprising a biological molecule; and
eluting the biological molecule from the coated device.

2. The method of claim 1, wherein the bodily fluid is selected from the group consisting of blood, serum, plasma, platelet rich plasma, bone marrow, cerebrospinal fluid, urine, synovial fluid, and combinations thereof.

3. The method of claim 1, wherein incubating the coated device with the bodily fluid comprises incubating the coated device with the bodily fluid for a time period of from less than one minute to about 120 minutes.

4. The method of claim 1, wherein eluting the biological molecule from the coated device comprises contacting the coated device with an ionic buffer selected from the group consisting of a phosphate buffer, a sodium chloride buffer, a magnesium chloride buffer, a calcium chloride buffer, a sodium fluoride buffer, and combinations thereof.

5. The method of claim 4, wherein the ionic buffer comprises up to 0.5 M phosphate.

6. The method of claim 4, wherein the ionic buffer comprises up to 0.2 M sodium chloride.

7. The method of claim 4, wherein the ionic buffer comprises up to 5.0 M magnesium chloride.

8. The method of claim 4, wherein the ionic buffer comprises up to 5.0 M calcium chloride.

9. The method of claim 4, wherein the ionic buffer comprises up to 0.4 M sodium fluoride.

10. The method of claim 1, wherein eluting the biological molecule from the coated device comprises contacting the coated device with a mineral dissolution buffer, wherein the mineral dissolution buffer is selected from the group consisting of phosphoric acid, ethylenediaminetetraacetic acid, hydrochloric acid, sodium hydroxide, and combinations thereof.

11. The method of claim 10, wherein the mineral dissolution buffer comprises up to 0.5 M phosphoric acid.

12. The method of claim 1, wherein the mineral coating is selected from the group consisting of calcium, phosphate, carbonate, and combinations thereof.

13. The method of claim 12, wherein the mineral coating is elected from the group consisting of hydroxyapatite (HAP), α-tricalcium phosphate (α-TCP), β3-tricalcium phosphate (β3-TCP), amorphous calcium phosphate, dicalcium phosophate, octacalcium phosphate, and calcium carbonate.

14. The method of claim 1, wherein the substrate comprises a poly(α-hydroxy ester) selected from the group consisting of poly(L-lactide), poly(lactide-co-glycolide), poly(ε-caprolactone), and combinations thereof.
